# EUROPEAN PATENT APPLICATION

(11) **EP 4 835 069 A1**
(43) Date of publication of application: **04.11.2026**
(21) Application number: 24913516.1
(22) Date of filing: 23.12.2024
(51) Int. Cl.: C12N 9/90, C12N 15/70, C12P 19/02, C12P 19/24

(54) **ALLULOSE 3-EPIMERASE VARIANT WITH IMPROVED THERMAL STABILITY AND USES THEREOF**

(30) Priority: 28.12.2023 KR 20230195105
(71) Applicant: Samyang Corporation, Jongno-gu Seoul 03129 (KR)
(72) Inventor: HEO, Jinsol, Seongnam-si, Gyeonggi-do 13479 (KR); KIM, Jeongmin, Yongin-si, Gyeonggi-do 16826 (KR); LEE, Sanghee, Gwangju-si, Gyeonggi-do 12788 (KR); PARK, Busoo, Hanam-si, Gyeonggi-do 13014 (KR); JO, Heegeun, Seoul 07052 (KR)
(74) Representative: Vossius & Partner Patentanwälte Rechtsanwälte mbB
(86) International application number: PCT/KR2024/020971
(87) International publication number: WO 2025/143723

(57) **Abstract**

The present disclosure relates to an enzyme protein including a mutated amino acid sequence and having an activity of converting fructose to allulose, a recombinant microorganism including a gene encoding the enzyme protein, a composition for producing allulose, including the enzyme protein, and a method of producing allulose, using the composition.

## Description

### Technical Field

The present disclosure provides an allulose 3-epimerase mutant protein with improved thermal stability, a recombinant microorganism and/or a composition for producing allulose, each including the same, and/or a method of producing allulose, using the same.

### Background Art

Glucose is an essential sugar used as an energy source when ingested by the body. However, excessive intake of glucose causes adult diseases such as obesity and diabetes. To overcome this, allulose (D-allulose, D-psicose) has recently emerged as a sugar material.

Allulose is produced by the epimerization of fructose (D-fructose) and has a low calorie content due to its structure that is difficult to use as an energy source upon ingestion. In this regard, allulose has a function of inhibiting the absorption of glucose, thereby preventing an increase in blood sugar, making it effective for diabetic diseases. In addition, allulose inhibits the action of enzymes involved in fat accumulation, thereby preventing fat accumulation and functioning as a sugar effective for obesity. Moreover, allulose can serve as a material that can be converted into allose having anticancer effects, and itself has also been reported to exhibit similar effects. Thus, allulose is a sugar material expected to find expanded use as a health-functional sugar.

Furthermore, with a sweetness level equivalent to 70% that of sucrose, allulose is a sugar capable of maintaining an appropriate level of sweetness upon consumption. Therefore, allulose has recently been widely used as a functional sugar and is extensively used in low-calorie beverages.

Since the functionality of allulose was confirmed, research has been ongoing to develop methods of producing allulose through the conversion of fructose. In addition, research is also underway on methods to produce allulose using glucose, which has a lower raw material cost than fructose. Regarding the conversion of fructose to allulose, research is underway on conversion using allulose epimerases derived from *Agrobacterium tumefaciense*, *Clostridium cellulolyticum*, and *Clostridium boleae*, tagatose epimerases derived from *Pseudomonas chichori* and *Rhodobacter sphaeroides*, and enzymes derived from microorganisms such a*s Arthrobacter gloformis*, *Staphylococcus aureus*, *Mesorhizobium loti*, and *Methylonmonus* sp. Recently, to reduce production costs, research has also been conducted on allulose production via multi-enzyme conversion using glucose or starch.

Generally, allulose is converted from fructose via epimerization of the hydroxyl moiety at the C-3 position of fructose. Generally, enzymatic reactions reach an equilibrium with a ratio of 70:30 between fructose and a substrate. Therefore, the conversion yield of allulose generally reaches a maximum of approximately 30 %.

However, numerous reports have confirmed that increasing the conversion reaction temperature leads to a higher conversion yield of allulose. Therefore, by securing the thermal stability of an enzyme to enhance the stability thereof, it is possible to increase the conversion yield by raising the conversion reaction temperature.

Accordingly, this study aimed to investigate enzyme mutants with improved thermal stability and identify those exhibiting both enhanced activity and thermal stability, thereby increasing allulose productivity.

### Disclosure of Invention

### Technical Problem

An objective of the present disclosure is to provide an allulose 3-epimerase mutant protein having improved thermal stability and allulose conversion activity, a polynucleotide encoding the allulose 3-epimerase mutant protein, a recombinant vector including the polynucleotide, or a recombinant microorganism including the polynucleotide or the recombinant vector.

Another objective of the present disclosure is to provide a composition for producing allulose, including one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

Another objective of the present disclosure is to provide a method of producing allulose, including reacting, with a substrate, one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

Another objective of the present disclosure is to provide use of a composition for producing allulose, the composition including one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

Another objective of the present disclosure is to provide use of the allulose 3-epimerase mutant protein, a recombinant microorganism expressing the allulose 3-epimerase mutant protein, cells of the microorganism, a lysate of the cells of the microorganism, a culture of the microorganism, or extracts thereof, for preparing a composition for producing allulose.

### Solution to Problem

An embodiment of the present disclosure provides an allulose 3-epimerase protein including an amino acid sequence having 80 % or more sequence identity with the amino acid sequence of SEQ ID NO: 1, the amino acid sequence including substitutions of amino acids at positions corresponding to some amino acids in the amino acid sequence of SEQ ID NO: 1.

Specifically, the allulose 3-epimerase protein has, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285
with one or more amino acids selected from the group consisting of leucine (L), asparagine (N), alanine (A), valine (V), glutamine (Q), glutamic acid (E), isoleucine (I), threonine (T), glycine (G), and proline (P).

Another embodiment of the present disclosure provides a polynucleotide encoding the allulose 3-epimerase protein.

Specifically, the present disclosure provides a polynucleotide encoding an enzyme protein, the enzyme protein including an amino acid sequence having 80 % or more sequence identity with the amino acid sequence of SEQ ID NO: 1, the amino acid sequence including, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285
with one or more amino acids selected from the group consisting of leucine (L), asparagine (N), alanine (A), valine (V), glutamine (Q), glutamic acid (E), isoleucine (I), threonine (T), glycine (G), and proline (P).

Another embodiment of the present disclosure provides a recombinant vector including the polynucleotide.

Another embodiment of the present disclosure provides a recombinant microorganism including the polynucleotide encoding the enzyme protein or a recombinant vector including the same.

The microorganism may be, but is not limited to, one or more microorganisms selected from the group consisting of strains of the genus *Escherichia* (*Escherichia coli*), strains of the genus *Bacillus* (e.g., *Bacillus subtilis*), strains of the genus *Corynebacterium* (e.g., *Corynebacterium glutamicum*), strains of the genus *Saccharomyces* (e.g., *Saccharomyces cerevisiae*), and strains of the genus *Pichia* (e.g., *Pichia pastoris*).

Another embodiment of the present disclosure provides a composition for producing allulose, including one or more selected from the group consisting of the enzyme protein, a recombinant microorganism expressing the enzyme protein, cells of the microorganism, a lysate of the cells of the microorganism, a culture of the microorganism, and extracts thereof.

Another embodiment of the present disclosure provides a method of producing allulose, including reacting, with a substrate, one or more selected from the group consisting of the enzyme protein, a recombinant microorganism expressing the enzyme protein, cells of the microorganism, a lysate of the cells of the microorganism, a culture of the microorganism, and extracts thereof.

The enzyme protein according to the present disclosure includes an amino acid sequence having 80 % or more sequence identity with the amino acid sequence of SEQ ID NO: 1, the amino acid sequence including, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285
with one or more amino acids selected from the group consisting of leucine (L), asparagine (N), alanine (A), valine (V), glutamine (Q), glutamic acid (E), isoleucine (I), threonine (T), glycine (G), and proline (P), and

the enzyme protein has improved thermal stability and allulose conversion activity as compared to an enzyme protein including an amino acid sequence having, from the N-terminus of the amino acid sequence of SEQ ID NO: 1, substitutions of amino acids at positions 23 and 267 with isoleucine (I) and lysine (K), respectively.

Hereinafter, the present disclosure will be described in more detail.

### Allulose 3-epimerase mutant protein

An embodiment of the present disclosure provides an allulose 3-epimerase protein including an amino acid sequence having 80 % or more sequence identity with the amino acid sequence of SEQ ID NO: 1, the amino acid sequence including, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285
with one or more amino acids selected from the group consisting of leucine (L), asparagine (N), alanine (A), valine (V), glutamine (Q), glutamic acid (E), isoleucine (I), threonine (T), glycine (G), and proline (P).

Specifically, the substituted amino acid sequence may include, from the N-terminus of the amino acid sequence of SEQ ID NO: 1,
(1) substitution of alanine at a position corresponding to position 23 with isoleucine and substitution of glutamic acid at a position corresponding to position 267 with lysine, and
(2) one or more selected from the group consisting of substitution of serine (S) at a position corresponding to position 67 with threonine, substitution of glycine at a position corresponding to position 68 with proline, substitution of proline at a position corresponding to position 77 with isoleucine, substitution of isoleucine at a position corresponding to position 106 with leucine, substitution of valine at a position corresponding to position 132 with leucine, substitution of serine at a position corresponding to position 142 with asparagine, substitution of serine at a position corresponding to position 142 with alanine, substitution of aspartame at a position corresponding to position 170 with glycine, substitution of leucine at a position corresponding to position 174 with valine, substitution of alanine at a position corresponding to position 192 with threonine, substitution of alanine at a position corresponding to position 192 with glutamine, substitution of glycine at a position corresponding to position 232 with glutamic acid, substitution of aspartame at a position corresponding to position 278 with glutamine, substitution of serine at a position corresponding to position 285 with glutamic acid, and substitution of serine at a position corresponding to position 285 with asparagine. However, the present disclosure is not limited thereto.

Specifically, the substituted amino acid sequence may include, from the N-terminus of the amino acid sequence of SEQ ID NO: 1,
(1) substitution of alanine at a position corresponding to position 23 with isoleucine and substitution of glutamic acid at a position corresponding to position 267 with lysine, and
(2) substitution of isoleucine at a position corresponding to position 106 with leucine, substitution of serine at a position corresponding to position 142 with asparagine, substitution of serine at a position corresponding to position 142 with alanine, substitution of leucine at a position corresponding to position 174 with valine, substitution of aspartame at a position corresponding to position 278 with glutamine, substitution of serine at a position corresponding to position 285 with glutamic acid, or substitution of serine at a position corresponding to position 285 with asparagine. However, the present disclosure is not limited thereto.

Specifically, the substituted amino acid sequence may include, from the N-terminus of the amino acid sequence of SEQ ID NO: 1,
(1) substitution of alanine at a position corresponding to position 23 with isoleucine, substitution of glutamic acid at a position corresponding to position 267 with lysine, and substitution of aspartame at a position corresponding to position 278 with glutamine, or substitution of alanine at a position corresponding to position 23 with isoleucine, substitution of glutamic acid at a position corresponding to position 267 with lysine, and substitution of serine at a position corresponding to position 285 with glutamic acid, and
(2) substitution of isoleucine at a position corresponding to position 106 with leucine, substitution of valine at a position corresponding to position 132 with leucine, substitution of serine at a position corresponding to position 142 with asparagine, substitution of aspartame at a position corresponding to position 170 with glycine, substitution of alanine at a position corresponding to position 192 with threonine, substitution of alanine at a position corresponding to position 192 with glutamine, or substitution of glycine at a position corresponding to position 232 with glutamic acid. However, the present disclosure is not limited thereto.

Specifically, the substituted amino acid sequence may include, from the N-terminus of the amino acid sequence of SEQ ID NO: 1,
(1) substitution of alanine at a position corresponding to position 23 with isoleucine, substitution of isoleucine at a position corresponding to position 106 with leucine, substitution of glutamic acid at a position corresponding to position 267 with lysine, and substitution of serine at a position corresponding to position 285 with glutamic acid, and
(2) substitution of serine (S) at a position corresponding to position 67 with threonine, substitution of glycine at a position corresponding to position 68 with proline, or substitution of serine at a position corresponding to position 142 with asparagine. However, the present disclosure is not limited thereto.

An embodiment of the present disclosure provides an allulose 3-epimerase protein including an amino acid sequence having 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 98.9 %, 99 %, 99.3 %, or 99.5 % or more sequence identity or homology with the amino acid sequence of SEQ ID NO: 1, the amino acid sequence having, from the N-terminus of the amino acid sequence of SEQ ID NO: 1, substitutions of amino acids at positions corresponding to positions 23 and 267 with isoleucine (I) and lysine (K), respectively.

Specifically, the allulose 3-epimerase protein provided by the present disclosure includes an amino acid sequence having 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 98.9 %, 99 %, 99.3 %, or 99.5 % or more sequence identity or homology with the amino acid sequence of SEQ ID NO: 1, the amino acid sequence including, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285

with one or more amino acids selected from the group consisting of leucine (L), asparagine (N), alanine (A), valine (V), glutamine (Q), glutamic acid (E), isoleucine (I), threonine (T), glycine (G), and proline (P), and
the allulose 3-epimerase protein may exclude an allulose 3-epimerase protein including an amino acid sequence having, in the amino acid sequence of SEQ ID NO: 1, substitution of an amino acid at position 23 with isoleucine (I), and substitution of an amino acid at position 267 with lysine (K), i.e., an allulose 3-epimerase protein having two or fewer mutations.

An embodiment of the present disclosure provides an allulose 3-epimerase protein including an amino acid sequence having 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 98.9 %, 99 %, 99.3 %, or 99.5 % sequence identity or homology with the amino acid sequence of SEQ ID NO: 1, the amino acid sequence including, from the N-terminus of the amino acid sequence of SEQ ID NO: 1,
(1) substitutions of amino acids at positions corresponding to positions 23 and 267 with isoleucine (I) and lysine (K), respectively, and
(2) substitution(s) of one or more amino acids selected from the group consisting of amino acids at positions corresponding to positions 67, 68, 77, 106, 132, 142, 170, 174, 192, 232, 278, and 285

with one or more amino acids selected from the group consisting of leucine (L), asparagine (N), alanine (A), valine (V), glutamine (Q), glutamic acid (E), isoleucine (I), threonine (T), glycine (G), and proline (P), and
the allulose 3-epimerase protein may exclude an allulose 3-epimerase protein including an amino acid sequence having, from the N-terminus of the amino acid sequence of SEQ ID NO: 1, substitution of an amino acid at position 23 with isoleucine (I), and substitution of an amino acid at position 267 with lysine (K), i.e., an allulose 3-epimerase protein having two or fewer mutations.

Comparison of identity and homology may be performed by calculating the percentage (%) of identity and homology between two or more sequences using a commercially available computer program.

It is obvious that a protein having an amino acid sequence that is partially deleted, modified, substituted, or added also falls within the scope of the present application, provided that the amino acid sequence has the identity or homology mentioned in the present disclosure and exhibits activity corresponding to that of the allulose 3-epimerase protein. That is, even if the present application describes a "protein having or including an amino acid sequence represented by a specific SEQ ID NO, it is obvious that a protein having (or including) an amino acid sequence that is partially deleted, modified, substituted, or added can also be used in the present application, provided that the protein has an activity identical or corresponding to that of the protein consisting of the amino acid sequence of the corresponding SEQ ID NO.

In the present disclosure, "corresponding amino acid" refers to an amino acid residue at a corresponding position in the allulose 3-epimerase protein, or an amino acid residue that is similar, identical, or homologous to the amino acid residue at a corresponding position. Identifying an amino acid at a corresponding position may involve determining a specific amino acid in a sequence with reference to a specific sequence. In the present disclosure, "corresponding position" generally refers to a similar or corresponding position in the amino acid sequence of a related protein or a reference sequence. For example, any amino acid sequence may be aligned with SEQ ID NO: 1, and based on this alignment, each amino acid residue of the amino acid sequence may be numbered with reference to the numerical positions of amino acid residues corresponding to the amino acid residues of SEQ ID NO: 1. For example, a position of a corresponding amino acid, or a position at which a modification such as substitution, insertion, or deletion occurs, may be identified through a sequence alignment algorithm known in the art by comparison with a query sequence (also referred to as a "reference sequence").

### Recombinant microorganism and composition for producing allulose

Another embodiment of the present disclosure provides a recombinant microorganism including a polynucleotide encoding the allulose 3-epimerase mutant protein or a recombinant vector including the same.

The recombinant microorganism may be, but is not limited to, one or more microorganisms selected from the group consisting of strains of the genus *Escherichia* (*Escherichia coli*), the genus *Bacillus* (e.g., *Bacillus subtilis*), the genus *Corynebacterium* (e.g., *Corynebacterium glutamicum*), the genus *Saccharomyces* (e.g., *Saccharomyces cerevisiae*), and the genus *Pichia* (e.g., *Pichia pastoris*).

Another embodiment of the present disclosure provides a composition for producing allulose, including one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

The culture may include an enzyme produced from the recombinant microorganism, and may be in the form of including the microorganism or in a cell-free form not including the microorganism. In other embodiments, the lysate includes a lysate obtained by lysing the cells of the recombinant microorganism or a supernatant obtained by centrifuging the lysate, and an enzyme produced from the recombinant microorganism.

In the present specification, unless otherwise stated, the recombinant microorganism used for the production of allulose is used to mean one or more selected from the group consisting of cells of the microorganism, a culture of the strain, and a lysate of the strain.

The activation of the allulose 3-epimerase mutant protein may be regulated by metal ions. Thus, in the production of allulose using the strain of the genus *Escherichia*, the conversion efficiency from fructose to allulose, i.e., the allulose production rate, may be increased by adding metal ions. Therefore, a composition for producing allulose, including one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof, or a composition for producing allulose using a microorganism producing the same, may further include metal ions. In other embodiments, a method of producing allulose using the allulose 3-epimerase mutant protein may further include adding metal ions.

The metal ions may be one or more selected from the group consisting of manganese ions, magnesium ions, nickel ions, cobalt ions, and the like. For example, the metal ions may be manganese ions, magnesium ions, nickel ions, cobalt ions, or a mixture thereof.

The amount of the metal ions added may be in a range of 0.5 mM to 5 mM, 0.5 mM to 4 mM, 0.5 mM to 3 mM, for example, 0.5 mM to 2 mM, in consideration of the effect of improving the allulose production yield.

### Allulose conversion activity and thermal stability

The allulose conversion activity in the enzymatic reaction of the allulose 3-epimerase mutant protein of the present disclosure may be measured using cells of a microorganism (recombinant microorganism) expressing allulose 3-epimerase, a culture medium in which the cells are cultured, and/or a supernatant obtained by centrifuging the culture medium. Specifically, the allulose conversion activity may be measured using cells obtained after reacting the cells with a substrate, a culture medium in which the cells are cultured, and/or a supernatant obtained by centrifuging the culture medium. The substrate may be, but is not limited to, one or more selected from the group consisting of fructose, allulose, tagatose, xylose, sorbose, ribulose, and ketose.

The cells used for identifying the allulose conversion activity in the enzymatic reaction of the allulose 3-epimerase mutant protein may be cells of a strain of the genus *Escherichia* (e.g., *Escherichia coli*), but the present disclosure is not limited thereto.

In a substrate conversion reaction using the allulose 3-epimerase mutant protein or cells of a microorganism producing the same, the allulose conversion activity of the allulose 3-epimerase mutant protein according to the present disclosure, or the allulose 3-epimerase mutant protein in the composition for producing allulose, may be 102 % or more, 105 % or more, 107 % or more, 110 % or more, 112 % or more, 115 % or more, 117 % or more, 120 % or more, 125 % or more, 130 % or more, 135 % or more, 140 % or more, 145 % or more, 150 % or more, 155 % or more, 160 % or more, 165 % or more, 170 % or more, 102 % to 200 %, 105 % to 200 %, 107 % to 200 %, 110 % to 200 %, 112 % to 200 %, 115 % to 200 %, 117 % to 200 %, 120 % to 200 %, 125 % to 200 %, 130 % to 200 %, 135 % to 200 %, 140 % to 200 %, 145 % to 200 %, 150 % to 200 %, 155 % to 200 %, 160 % to 200 %, 165 % to 200 %, or 170 % to 200 %, for example, 105 %, 110 %, 115 %, 120 %, 130 %, 135 %, 150 %, or 170 %, with respect to 100 % of the allulose conversion activity of an allulose 3-epimerase mutant protein including an amino acid sequence having, from the N-terminus of the amino acid sequence of SEQ ID NO: 1, substitution of an amino acid at position 23 with isoleucine (I), and substitution of an amino acid at position 267 with lysine (K), but the present disclosure is not limited thereto. The enzyme activity evaluation using cells of a microorganism producing allulose 3-epimerase or the allulose 3-epimerase mutant protein may be performed by carrying out a substrate conversion reaction at 70 °C for 30 minutes using cells of a microorganism expressing the allulose 3-epimerase mutant protein, or a substrate conversion reaction at 70 °C for 30 minutes using a purified enzyme obtained by lysing the cells of a microorganism producing allulose 3-epimerase.

The thermal stability of the allulose 3-epimerase mutant protein (the microorganism expressing the allulose 3-epimerase mutant protein) of the present disclosure or the composition for producing allulose of the present disclosure may be confirmed by measuring the half-life of the allulose 3-epimerase mutant protein, which is the point at which the activity reaches 50 % compared to 100 % of the allulose conversion activity before heat treatment, after heat-treating the cells of the microorganism (recombinant microorganism) expressing the allulose 3-epimerase mutant protein, a culture medium in which the cells are cultured, and/or a supernatant obtained by centrifuging the culture medium, but the present disclosure is not limited thereto.

The cells used for identifying the allulose conversion activity in the enzymatic reaction of the allulose 3-epimerase mutant protein may be cells of a strain of the genus *Escherichia* (e.g., *Escherichia coli*), but the present disclosure is not limited thereto.

The identification of the thermal stability may be performed by carrying out heat treatment at 80 °C for 30 minutes, 1 hour, 90 minutes, 3 hours, 5 hours, or 7 hours, using the cells of a microorganism expressing the allulose 3-epimerase mutant protein or a purified enzyme obtained by lysing the cells of a microorganism expressing the allulose 3-epimerase mutant protein.

The half-life (hr) of the allulose 3-epimerase mutant protein of the present disclosure or the allulose 3-epimerase mutant protein in the composition for producing allulose of the present disclosure under a heat treatment condition of 80 °C may be 600 % or more, 800 % or more, 1,000 % or more, 1,200 % or more, 1,400 % or more, 1,600 % or more, 1,800 % or more, 2,000 % or more, 2,200 % or more, 2,400 % or more, 2,600 % or more, 2,800 % or more, 3,000 % or more, 3,200 % or more, 3,400 % or more, 3,600 % or more, 3,800 % or more, 4,000 % or more, 600 % to 5,000 %, 800 % to 5,000 %, 1,000 % to 5,000 %, 1,200 % to 5,000 %, 1,400 % to 5,000 %, 1,600 % to 5,000 %, 1,800 % to 5,000 %, 2,000 % to 5,000 %, 2,200 % to 5,000 %, 2,400 % to 5,000 %, 2,600 % to 5,000 %, 2,800 % to 5,000 %, 3,000 % to 5,000 %, 3,200 % to 5,000 %, 3,400 % to 5,000 %, 3,600 % to 5,000 %, 3,800 % to 5,000 %, or 4,000 % to 5,000 %, for example, about 621 %, 675 %, 756 %, 810 %, 972 %, 1069.2 %, 1080 %, 1458 %, 1836 %, 1944 %, 2430 %, 2478.6 %, 2937.6 %, 3304.8 %, 3855.6 %, or 4039.2, with respect to 100 % of the half-life of an allulose 3-epimerase protein including the amino acid sequence of SEQ ID NO: 1, but the present disclosure is not limited thereto.

The half-life (hr) of the allulose 3-epimerase mutant protein of the present disclosure or the allulose 3-epimerase mutant protein in the composition for producing allulose of the present disclosure under a heat treatment condition of 80 °C may be 110 % or more, 120 % or more, 130 % or more, 140 % or more, 150 % or more, 160 % or more, 170 % or more, 180 % or more, 190 % or more, 200 % or more, 250 % or more, 300 % or more, 400 % or more, 450 % or more, 500 % or more, 550 % or more, 600 % or more, 650 % or more, 700 % or more, 110 % to 800 %, 120 % to 800 %, 130 % to 800 %, 140 % to 800 %, 150 % to 800 %, 160 % to 800 %, 170 % to 800 %, 180 % to 800 %, 190 % to 800 %, 200 % to 800 %, 250 % to 800 %, 300 % to 800 %, 400 % to 800 %, 450 % to 800 %, 500 % to 800 %, 550 % to 800 %, 600 % to 800 %, 650 % to 800 %, or 700 % to 800 %, for example, 115 %, 125 %, 140 %, 150 %, 180 %, 198 %, 200 %, 270 %, 340 %, 360 %, 450 %, 459 %, 544 %, 612 %, 714 %, or 748 %, with respect to 100 % of the half-life of an allulose 3-epimerase mutant protein including an amino acid sequence having, from the N-terminus of the amino acid sequence of SEQ ID NO:1, substitution of an amino acid at position 23 with isoleucine (I),and substitution of an amino acid at position 267 with lysine (K), but the present disclosure is not limited thereto.

### Method of producing allulose

Another embodiment of the present disclosure provides a method of producing allulose, including reacting, with a substrate, one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

The substrate may be, but is not limited to, one or more selected from the group consisting of fructose, allulose, tagatose, xylose, sorbose, ribulose, and ketose.

The reaction may include, but is not limited to, bringing a medium including one or more selected from the group consisting of fructose, allulose, tagatose, xylose, sorbose, ribulose, and ketose, into contact with one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

Another objective of the present disclosure provides use of a composition for producing allulose, the composition including one or more selected from the group consisting of the allulose 3-epimerase mutant protein, a recombinant microorganism expressing the allulose 3-epimerase mutant protein, cells of the microorganism, a lysate of the cells of the microorganism, a culture of the microorganism, and extracts thereof.

Another objective of the present disclosure provides use of the allulose 3-epimerase mutant protein, a recombinant microorganism expressing the allulose 3-epimerase mutant protein, cells of the microorganism, a lysate of the cells of the microorganism, a culture of the microorganism, or extracts thereof, for preparing a composition for producing allulose.

The allulose 3-epimerase mutant protein, recombinant microorganism, cells of the microorganism, a lysate of the cells of the microorganism, culture of the microorganism, extracts thereof, allulose production, etc. are as described above.

### Advantageous Effects of Invention

The present disclosure relates to an enzyme protein having allulose 3-epimerization activity, in which specific amino acids are mutated, a recombinant microorganism including the same, a composition for producing allulose, or a method of producing allulose, using the same, the enzyme protein having high conversion activity from fructose to allulose and exhibiting excellent thermal stability.

### Brief Description of the Drawings

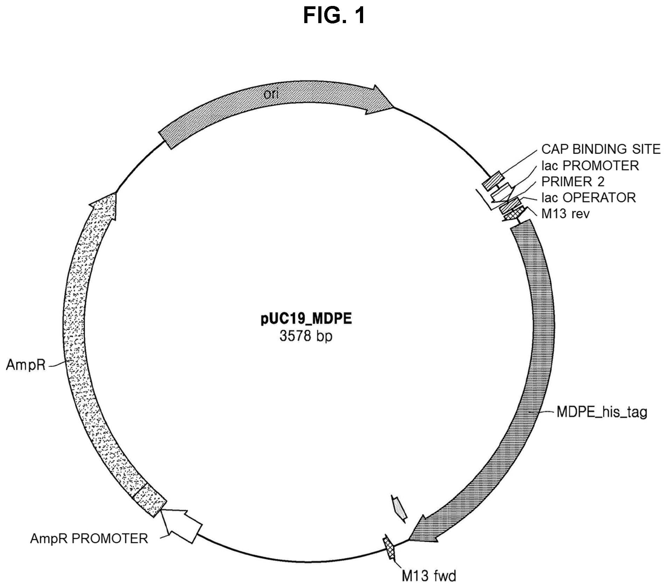

FIG. 1 is a schematic view of a recombinant vector into which a D-allulose 3-epimerase gene is cloned, according to an embodiment of the present disclosure.

### Mode for the Invention

The present disclosure will be described in further detail with reference to the following examples. However, these examples are not intended to limit the scope of the present disclosure.

### Example 1. Cloning of D-allulose 3-epimerase gene

### Example 1-1. Preparation of enzyme gene

D-allulose 3-epimerase identified in *Microbacterium foliorum* (SY27B-MF; accession number: KCCM11774P) was expressed in *E. coli* to confirm enzyme activity, and a DNA sequence was synthesized to identify mutants with improved thermal stability. The amino acid sequence of the protein was synthesized using codons optimized for *E. coli* to ensure proper expression in *E. coli*.

The gene sequence obtained through codon optimization was synthesized by Integrated DNA Technologies (IDT, USA). The synthesized gene was named MDPE, and information on the codon-optimized gene is shown in Table 1. The amino acid sequence of the MDPE gene corresponds to the sequence of SEQ ID NO: 1, and the nucleotide sequence corresponds to the sequence of SEQ ID NO: 2.

**Table 1**

| Type | Sequence (5' -> 3') | SEQ ID NO: |
|---|---|---|
| MDPE_Wild_AA | MNIGCHGLVWTGHFDADGIRLSAEQTKAAGFDLIEFPL MDPFTFDVAAAKEALEEHDLAVSASLGLSGATDVTSSD PAVVAAGEALLMKAVDVLAELGGRHFCGVIYSAMQKY MDPVTTEGLESSRRTIARVADHAAERGISVSLEVVNRY ETNVLNTARQALAYLAEVDRPNLGIHLDTYHMNIEESD MFAPVLDAAPALRYVHIGESHRGYLGTGTVDFDNFFKA LGRIGYDGPIVFESFSSAVVAPDLSRMLGIWRNLWTDN VELGAHANAYIRDKLVAVDSIRLH | 1 |
| MDPE_Wild_DN A | ATGAACATCGGTTGCCACGGTCTGGTTTGGACCGGT CACTTCGACGCGGACGGTATCCGTCTGTCTGCGGAA CAGACCAAAGCGGCGGGTTTCGACCTGATCGAATTC CCGCTGATGGACCCGTTCACCTTCGACGTTGCGGC GGCGAAAGAAGCGCTGGAAGAACACGACCTGGCGG TTTCTGCGTCTCTGGGTCTGTCTGGTGCGACCGACG TTACCTCTTCTGACCCGGCGGTTGTTGCGGCGGGTG AAGCGCTGCTGATGAAAGCGGTTGACGTTCTGGCG GAACTGGGTGGTCGTCACTTCTGCGGTGTTATCTAC TCTGCGATGCAGAAATACATGGACCCGGTTACCACC GAAGGTCTGGAATCTTCTCGTCGTACCATCGCGCGT GTTGCGGACCACGCGGCGGAACGTGGTATCTCTGTT TCTCTGGAAGTTGTTAACCGTTACGAAACCAACGTTC TGAACACCGCGCGTCAGGCGCTGGCGTACCTGGCG GAAGTTGACCGTCCGAACCTGGGTATCCACCTGGAC ACCTACCACATGAACATCGAAGAATCTGACATGTTCG CGCCGGTTCTGGACGCGGCGCCGGCGCTGCGTTAC GTTCACATCGGTGAATCTCACCGTGGTTACCTGGGT ACCGGTACCGTTGACTTCGACAACTTCTTCAAAGCG CTGGGTCGTATCGGTTACGACGGTCCGATCGTTTTC GAATCTTTCTCTTCTGCGGTTGTTGCGCCGGACCTG TCTCGTATGCTGGGTATCTGGCGTAACCTGTGGACC GACAACGTTGAACTGGGTGCGCACGCGAACGCGTA CATCCGTGACAAACTGGTTGCGGTTGACTCTATCCG | 2 |
| | TCTGCAC | |

### Example 1-2. Construction of vector including enzyme gene

The MDPE polynucleotide of Example 1-1, synthesized through codon optimization for expression in *E. coli*, was amplified by polymerase chain reaction (PCR). Specifically, a gene amplification (polymerase chain reaction, PCR) reaction solution with a final volume of 50 µl was prepared, including 50 ng of the synthesized MDPE gene, 10 pmol of a forward primer (nucleotide sequence of SEQ ID NO: 3) and 10 pmol of a reverse primer (nucleotide sequence of SEQ ID NO: 4) for each amino acid residue, 1 µl of Phusion polymerase, 10 µl of reaction buffer (5X), and 1 µl of 10 mM dNTP, and then amplified by PCR. The sequence information of the primers used is shown in Table 2.

**Table 2**

| Type | Nucleotide sequence (5' -> 3') | SEQ ID NO: |
|---|---|---|
| MDPE_Cloning_For_Primer | CCATGATTACGCCAAGCTTATGAAC ATCGGTTGCCACG | 3 |
| MDPE_Cloning_Rev_Primer | CGGTACCCGGGGATCCTAGCAGCC GGATCTCAGTGCAGACGGATAGAG TC | 4 |

Specifically, the PCR was performed using the PCR solution in GeneAmp PCR system 9700 under the following conditions: one cycle at 98 °C for 30 seconds; 18 cycles at 98 °C for 10 seconds, 55 °C for 30 seconds, and 72 °C for 2.5 minutes; and one cycle at 72 °C for 5 minutes to amplify the gene, thereby obtaining a large amount of the gene.

The pUC19 vector (NEB, New England Biolab) was digested with the restriction enzymes *HindIII* and *BamHI*. The amplified gene was then inserted and ligated into the restriction sites of the pUC19 vector using 2XHiFi DNA master mix (NEB) to construct the pUC19/allulose 3-epimerase recombinant vector (pUC19_MDPE). A schematic view of the constructed recombinant vector is illustrated in FIG. 1. The constructed recombinant vector was transformed into *E. coli* DH10b competent cells (TRANS, Trans10) using a heat shock method to produce a recombinant microorganism (DH10b pUC19_MDPE).

### Example 2. Preparation of primary mutant enzymes

Based on amino acid sequence analysis of homologous genes and 3D structure model analysis of active and metal-binding sites, selected amino acids were mutated by substitution with other amino acids through site-directed mutagenesis (SDM) and saturation mutagenesis.

### Example 2-1. Site-directed mutagenesis (SDM)

Homologous regions were identified by analyzing D-allulose 3-epimerases derived from *Agrobacterium tumefaciense, Clostridium cellulolyticum* and *Methylomonus sp.,* and the sequences thereof, which had been identified among epimerases, and mutation sites were determined based on the homologous regions.

Specifically, as a result of comparing the homologies of the epimerases, it was confirmed that the amino acid at position 23 in the D-allulose 3-epimerases derived from *Agrobacterium tumefaciense*, *Clostridium cellulolyticum*, and *Methylomonus* sp. was isoleucine (I), which was then selected as a mutation site. It was confirmed using the Protein Data bank database that the structural activities of the enzymes were maintained at the selected position.

A substitution mutant of MDPE of Example 1 was constructed via site-directed mutagenesis (SDM) using a Quikchange^{®} (Stratagene), which has been applied in conventional studies. The Stratagene Quikchange^{®} site-directed mutagenesis method does not involve amplifying only the gene corresponding to the enzyme and subcloning the same into a plasmid DNA, but rather involves amplifying the entire plasmid DNA via PCR and digesting the template DNA with the DpnI enzyme. The Quikchange^{®} site-directed mutagenesis method has the advantage of being able to induce mutations more rapidly.

pUC19_MDPE of Example 1-2 was used as a template DNA for replication to introduce the substitution mutations of MDPE of Example 1. A Polymerase Chain Reaction (PCR) mixture with a final volume of 50 µl was prepared, containing 10 ng of pUC19_MDPE, 10 pmol of a forward primer (nucleotide sequence of SEQ ID NO: 5) and 10 pmol of a reverse primer (nucleotide sequence of SEQ ID NO: 6) for amino acid residue (I23), 1 µl of Phusion polymerase, 10 µl of reaction buffer (5X), and 1 µl of 10 mM dNTP, and was amplified via PCR. The information of the primers used is shown in Table 3.

**Table 3**

| Type | Nucleotide sequence (5' -> 3') | SEQ ID NO: |
|---|---|---|
| MDPE_A23I_Cloning_For_Primer | gacggtatccgtctgtctattgaacagacc aaagcggc | 5 |
| MDPE_A23I_Cloning_Rev_Primer | gccgctttggtctgttcaatagacagacgg ataccgtc | 6 |
| MDPE_Mut_For_Primer | gaccatgattacgccaagcttatg | 7 |
| MDPE_Mut_Rev_Primer | cggtacccggggatcctagcagccggat ctcagtg | 8 |

Specifically, the PCR was performed using the PCR mixture in GeneAmp PCR system 9700 under the following conditions: one cycle at 98 °C for 30 seconds; 18 cycles at 98 °C for 10 seconds, 55 °C for 30 seconds, and 72 °C for 2.5 minutes; and one cycle at 72 °C for 5 minutes to amplify the gene, thereby obtaining a large amount of the gene.

To 50 µl of the PCR mixture after completion of the reaction, 1 µl of DpnI (10 Unit/µl, NEB), which recognizes and digests non-mutated template DNA, was added, followed by incubation at 37 °C for 3 hours. After the incubation, the gene pUC19_MDPE_A23I (A23I mutation), which was amplified via PCR using MDPE_A23I_Cloning primers, was transformed into *E. coli* DH10b in substantially the same manner as the method of preparing the recombinant strain DH10b pUC19_MDPE of Example 1-2, thereby obtaining recombinant microorganism DH10b pUC19_MDPE_A23I.

To confirm the enzyme mutations within the prepared recombinant strain, a comparison was performed using the NCBI BLAST tool and sequence alignment with the amino acid sequence of the wild-type enzyme.

### Example 2-2. Mutagenesis via error-prone PCR

Mutants were prepared via error-prone PCR, which induces mutations during the gene amplification process. Specifically, to construct mutants, pUC19_MDPE_A23I prepared in Example 2-1 was used as a template DNA. A Polymerase Chain Reaction (PCR) mixture with a final volume of 50 µl was prepared by adding 10 ng of the template DNA, 10 pmol of a forward primer (nucleotide sequence of SEQ ID NO: 9) and 10 pmol of a reverse primer (nucleotide sequence of SEQ ID NO: 10) for the amino acid residue, 1 µl of Taq polymerase (Clontech), 5 µl of reaction buffer (10X TITANIUM Taq Buffer), 1 µl of diversify dNTP Mix, 1 µl of dGTP (2 mM), 4 µl of MnSO₄, and PCR-grade water, followed by amplification via PCR. The information of the primers used is shown in Table 3.

Specifically, the PCR mixture was used in GeneAmp PCR system 9700 with one cycle at 94 °C for 30 seconds, followed by 25 cycles at 94 °C for 30 seconds and at 68 °C for 1 minute, and a reaction at 68 °C for 1 minute.

The gene amplified through mutagenesis was obtained as a band at a desired position through electrophoresis, the obtained gene band was extracted, and the PCR-amplified gene was transformed into *E. coli* DH10b in substantially the same manner as the method of producing the recombinant microorganism DH10b pUC19_MDPE of Example 1-2, thereby obtaining a recombinant microorganism.

### Example 2-3. Screening of mutants using High-Throughput-Screening (HTS)

The transformed recombinant microorganism of Example 2-2 was spread on an LB ampicilline solid medium consisting of 10 g/L of Bacto Tryptone (Difco), 5 g/L of Yeast Extract (Difco), 10 g/L of NaCl (Daejung Chemicals & Metals), 100 mg/L of Ampicilline (Sigma), and 20 g/L of Bactor Agar to obtain colonies. The obtained colonies were subjected to primary screening using HTS.

After the primary screening, the colonies were inoculated into 300 uL of an LB-ampicilline liquid medium (consisting of 10 g/L of Bacto Tryptone (Difco), 5 g/L of Yeast Extract (Difco), 10 g/L of NaCl (Daejung Chemicals & Metals), and 100 mg/L of Ampicilline (Sigma)) in a 96-well (Greiner) multi-colony incubator, and then shake-cultured at 37 °C and 200 rpm for 16 to 18 hours.

The enzymatic activity of the shake-cultured cells was confirmed through a fructose-dehydrogenase assay. As for the method, 75 µL of the culture broth obtained by the shake-culture was transferred to a 96-well plate, and then an equal volume of a 100 mM allulose substrate (100 mM Mcilvaine buffer, pH 6.0) was added thereto, followed by performing a cell reaction at 60 °C for 30 minutes.

10 µL of the reaction mixture, in which fructose was produced from allulose through the reaction, was transferred to a new 96-well plate, and then solution A (150 mM Mcilvain buffer pH 4.5), solution B (0.1 M potassium ferricyanide, 0.1 % Triton X-100), and solution C (0.1 M potassium ferricyanide, 0.1 % Triton X-100, 0.05 % BSA) were mixed in a volume ratio of 70:10:10, and then 90 µL of the mixture was added to the reaction mixture converted to fructose.

Subsequently, after incubation at 37 °C for 30 minutes, 50 µL of solution D (1.25 mM Iron(III) sulfate hydrate, 0.3 % SDS, 8 % phosphoric acid) was added thereto, and then the reaction was stopped by incubation at 37 °C for 20 minutes. After the termination of the reaction, the absorbance of the reaction was measured at 660 nm, and strains with a higher absorbance were selected by comparison with the recombinant strain DH10b pUC19_MDPE of Example 1-2 in which enzyme mutation did not occur.

### Examples 2-4. Identification of mutated amino acid residues of mutant protein

By identifying the amino acid residue mutations of the enzyme in the recombinant microorganisms selected in Example 2-3, microorganisms in which A23I and E267K mutations were identified were selected.

Specifically, plasmids were purified and obtained from the recombinant microorganisms obtained in Example 2-3 using a plasmid prep kit (GENALL). Then, the gene sequence of the subcloned epimerase portion of the plasmid was transcribed into an amino acid sequence through a sequencing service (Macrogen) and compared with the amino acid sequence of the wild-type enzyme (SEQ ID NO: 1) to identify mutation sites. Information on the primers used therein is shown in Table 4.

**Table 4**

| Type | Sequence (5' -> 3') | SEQ ID NO: |
|---|---|---|
| M13F_Primer | GCGGATAACAATTTCACACAGG | 9 |
| M13R_Primer | GTAAAACGACGGCCAGT | 10 |

According to the method described above, microorganisms including an enzyme in which A23I and E267K mutations were identified among enzymes in the recombinant microorganisms were selected, and the selected DH10b pUC19_MDPE_A23I/E267K (A23I and E267K mutations) was prepared.

The positions of the amino acids mutated through the reaction were identified by generating a 3D structure of the enzyme through protein 3D structure homology modeling using SWISS-MODEL (http://www.expasy.org/swissmod/SWIIS-MODEL.html) and searching for a template in the Protein Data Bank (PDB), and the morphology of the 3D structure was confirmed.

### Example 3. Preparation of secondary mutant enzymes

### Example 3-1. Site-directed mutagenesis (SDM)

To induce an additional mutation by using the plasmid of DH10b pUC19_MDPE_A23I/E267K prepared in Example 2-4 as a template DNA, the SDM method of Example 2-1 was used.

Specifically, for DH10b pUC19_MDPE_A23I/E267K prepared in Examples 2-4, the purified and obtained plasmid pUC19_MDPE_A23I/E267K was prepared using a plasmid prep kit (GENALL) in substantially the same manner as in Example 2-4. The pUC19_MDPE_A23I/E267K prepared above was used as a template DNA, and a PCR mixture with a final volume of 50 µL was prepared, containing 10 ng of the template DNA, 10 pmol of a forward primer (nucleotide sequence of SEQ ID NO: 11, 13, 15, 17, 19, 21, or 23) and 10 pmol of a reverse primer (nucleotide sequence of SEQ ID NO: 12, 14, 16, 18, 20, 22, or 24) for each amino acid residue, 1 µL of Phusion polymerase, 10 µL of reaction buffer (5X), and 4 µL of 10 mM dNTP. Then, amplification was performed by PCR, and information on the primers used is shown in Table 5.

**Table 5**

| Type | Nucleotide sequence (5' -> 3') | SEQ ID NO: |
|---|---|---|
| MDPE_I106L_F | GTCGTCACTTCTGCGGTGTTTTGTACTCTGCGAT GCAGAAATAC | 11 |
| MDPE_I106L_R | GTATTTCTGCATCGCAGAGTACAAAACACCGCAG AAGTGACGAC | 12 |
| MDPE_V132L_F | CGTCGTACCATCGCGCGTCTGGCGGACCACGCG GCGG | 13 |
| MDPE_V132L_R | CCGCCGCGTGGTCCGCCAGACGCGCGATGGTAC GACG | 14 |
| MDPE_A192T_F | CATCGAAGAATCTGACATGTTCACCCCGGTTCTG GACGCGGC | 15 |
| MDPE_A192T_R | GCCGCGTCCAGAACCGGGGTGAACATGTCAGAT TCTTCGATG | 16 |
| MDPE_G232E_F | CAAAGCGCTGGGTCGTATCGAATACGACGGTCC GATCGTTTTC | 17 |
| MDPE_G232E_R | GAAAACGATCGGACCGTCGTATTCGATACGACCC AGCGCTTTG | 18 |
| MDPE_D170G_F | GCGTACCTGGCGGAAGTTGGCCGTCCGAACCTG GGTATC | 19 |
| MDPE_D170G_R | GATACCCAGGTTCGGACGGCCAACTTCCGCCAG GTACGC | 20 |
| MDPE_L174V_F | GGAAGTTGACCGTCCGAACGTGGGTATCCACCTG GACACC | 21 |
| MDPE_L174V_R | GGTGTCCAGGTGGATACCCACGTTCGGACGGTC AACTTCC | 22 |
| MDPE_D278Q_F | CGAACGCGTACATCCGTCAGAAACTGGTTGCGGT TGACTC | 23 |
| MDPE_D278Q_R | GAGTCAACCGCAACCAGTTTCTGACGGATGTACG CGTTCG | 24 |

(In Table 5, the underlined portions represent the nucleotide sequence portions corresponding to the amino acids to be substituted.)

Specifically, the PCR was performed using the PCR mixture in Takara PCR Thermal Cycler Dice^{TM} Touch under the following conditions: one cycle at 98 °C for 30 seconds; 25 cycles at 98 °C for 10 seconds, 55 °C for 30 seconds, and 72 °C for 2.5 minutes; and one cycle at 72 °C for 5 minutes to amplify the gene, thereby obtaining a large amount of the gene.

To 10 µl of the 50 µl PCR mixture after completion of the reaction, 0.5 µl of DpnI (10 Unit/µl, NEB), which recognizes and digests non-mutated template DNA, was added, followed by incubation at 37 °C for 3 hours. After the incubation, each of the resulting products was transformed into *E. coli* DH10b in substantially the same manner as the method of preparing a recombinant strain of Example 1-2 to obtain recombinant strains.

To confirm the enzyme mutations in the prepared recombinant strains, plasmids were extracted, and the gene sequences of the MDPE enzymes were sequenced and then compared with the wild-type enzyme using the NCBI website (BLAST) tool and amino acid sequence alignment of the wild-type enzyme.

### Example 3-2. Saturation mutagenesis

Saturation mutagenesis is an experimental method which is the same as site-directed mutagenesis (SDM), but differs in primer design to substitute amino acids at specific positions with various unspecified amino acids, rather than with other predetermined amino acids.

Specifically, a recombinant strain was obtained by inducing mutations in substantially the same manner as in Example 3-1, except for using the forward primer (nucleotide sequence of SEQ ID NO: 25 or 27) and the reverse primer (nucleotide sequence of SEQ ID NO: 26 or 28) of Table 6.

The primers used in saturation mutagenesis were constructed by incorporating codons of unspecified amino acids, such as NNN, as shown in the codons underlined in Table 6.

**Table 6**

| Type | Nucleotide sequence (5' -> 3') | SEQ ID NO: |
|---|---|---|
| MDPE_S142_F | cgcggcggaacgtggtatcrmngtttctctggaagttgttaacc | 25 |
| MDPE_S142_R | ggttaacaacttccagagaaacnkygataccacgttccgccgcg | 26 |
| MDPE_S285_F | caaactggttgcggttgacranatccgtctgcacccccgg | 27 |
| MDPE_S285_R | ccggggggtgcagacggatntygtcaaccgcaaccagtttg | 28 |

### Example 3-3. Identification of mutated amino acid residues in mutant

The amino acid residue mutations were identified in the recombinant microorganism obtained in Example 3-1 and the recombinant microorganism obtained through saturation mutagenesis in Example 3-2.

Specifically, plasmids were purified and obtained from the recombinant microorganisms of Examples 3-1 and 3-2 using a plasmid prep kit (GENALL). Subsequently, the gene sequences of the subcloned epimerase portions of the plasmids were transcribed into the amino acid sequence of the gene sequence through a sequence analysis service (Macrogen), and then mutation sites were identified by comparing these sequences with the wild-type amino acid sequence using the primers shown in Table 4.

The positions of the amino acids mutated through the reaction were identified by generating a 3D structure of the enzyme through protein 3D structure homology modeling using SWISS-MODEL (http://www.expasy.org/swissmod/SWIIS-MODEL.html) and searching for a template in the Protein Data Bank (PDB), and the morphology of the 3D structure was confirmed.

Accordingly, DH10b pUC19_MDPE_IK_I106L, DH10b pUC19_MDPE_IK_S142N, DH10b pUC19_MDPE_IK_S142A, DH10b pUC19_MDPE_IK_L174V, DH10b pUC19_MDPE_IK_D278Q (DH10b pUC19_MDPE_IKQ), DH10b pUC19_MDPE_IK_S285E (DH10b pUC19_MDPE_IKE), or DH10b pUC19_MDPE_IK_S285N was obtained through selection as a microorganism including an enzyme with identified I106L, S142N, S142A, L174V, D278Q, S285E, or S285N mutation, in addition to A23I and E267K mutations.

### Example 4. Evaluation of thermal stability of strains producing secondary mutant enzymes

Thermal stability was examined for the microorganism DH10b pUC19_MDPE_IK_I106L, DH10b pUC19_MDPE_IK_S142N, DH10b pUC19_MDPE_IK_S142A, DH10b pUC19_MDPE_IK_L174V, DH10b pUC19_MDPE_IK_D278Q (DH10b pUC19_MDPE_IKQ), DH10b pUC19_MDPE_IK_S285E (DH10b pUC19_MDPE_IKE), or DH10b pUC19_MDPE_IK_S285N, which were selected as microorganisms including an enzyme with the identified I106L, S142N, S142A, L174V, D278Q, S285E, or S285N mutation in addition to the A23I and E267K mutations in Example 3-3.

To confirm the thermal stability, DH10b pUC19_MDPE_A23I/E267K (DH10b pUC19_MDPE_IK) of Example 2-4, a microorganism including an enzyme with the identified A23I and E267K mutations, was used as a control.

Specifically, the recombinant microorganisms were each spread on an LB ampicilline solid medium consisting of 10 g/L of Bacto Tryptone (Difco), 5 g/L of Yeast Extract (Difco), 10 g/L of NaCl (Daejung Chemicals & Metals), 100 mg/L of Ampicilline (Sigma), and 20 g/L of Bactor Agar, and then cultured. A single colony was taken from the resulting solid culture and inoculated into 3 ml of LB ampicilline liquid medium (consisting of 10 g/L of Bacto Tryptone (Difco), 5 g/L of Yeast Extract (Difco), 10 g/L of NaCl (Daejung Chemicals & Metals), and 100 mg/L of Ampicilline (Sigma)), followed by shaking culture at 37 °C and 250 rpm for 16 to 18 hours. After the culture, cells of the microorganisms were harvested at an OD600nm of 2 to 3.

The harvested cells were diluted with 50 mM PIPES buffer (pH 6.0) containing 1 mM manganese ions to a cell concentration of 1 mg (dcw)/mL, followed by heat treatment at 80 °C for 30 minutes, 1 hour, 90 minutes, 3 hours, 5 hours, or 7 hours. Thereafter, a fructose substrate was added to a final concentration of 400 g/L, and a conversion reaction from fructose to allulose was performed at 70 °C for 30 minutes.

After the conversion reaction, the mixture was centrifuged (13,000 rpm, 10 min) to recover the supernatant, and high-performance liquid chromatography (HPLC) analysis was performed. The HPLC analysis was performed using a refractive index detector (RID, Agilent 1280 RID) of HPLC (Agilent, USA) equipped with a Cosmosil Sugar D column. 80 % (v/v) acetonitrile was used as a mobile phase solvent, and the temperature and flow rate were set to 35 °C and 1.0 mL/min, respectively. Based on the conversion activity measured over time through the HPLC analysis, the levels of thermal stability of the mutants were confirmed by calculating the half-life of activity relative to 100 % activity before the heat treatment, and the half-life was measured as the time point at which the activity of the mutants reached 50 %. The results thereof are shown in Table 7.

The values shown in Table 7 are calculated as percentages (%) of the half-life of each microorganism relative to the half-life of the microorganism DH10b pUC19_MDPE_IK at 80 °C, which is taken as 100 %.

**Table 7**

| Strain | Relative thermal stability value (%) |
|---|---|
| DH10b pUC19_MDPE_IK | 100 |
| DH10b pUC19_MDPE_IK_I106L | 150 |
| DH10b pUC19_MDPE_IK_S142N | 115 |
| DH10b pUC19_MDPE_IK_S142A | 125 |
| DH10b pUC19_MDPE_IK_L174V | 140 |
| DH10b pUC19_MDPE_IK_D278Q (IKQ) | 180 |
| DH10b pUC19_MDPE_IK_S285E (IKE) | 340 |
| DH10b pUC19_MDPE_IK_S285N | 200 |

As a result of measuring the half-life, it was confirmed that the microorganisms with additional mutations exhibited increased half-lives compared to the microorganism having the double mutations of A23I and E267K, indicating improved thermal stability. In particular, the thermal stability of microorganisms further having D278Q, S285E, or S285N mutation was found to be significantly increased.

### Example 5. Preparation of tertiary mutant enzyme and evaluation of thermal stability (1)

### Example 5-1. Construction and screening of additional mutants of D-allulose 3-epimerase in microorganism DH10b pUC19_MDPE_IKQ

Mutagenesis was performed through site-directed mutagenesis (SDM) using, as a template DNA, the selected MDPE_IKQ of Example 4, including an enzyme with excellent thermal stability. In this process, an additional mutation is introduced into the primary mutant, MDPE_IK_D278Q, through which the effect of double mutations can be confirmed.

Specifically, for DH10b pUC19_MDPE_IKQ prepared in Example 4, the purified and obtained plasmid pUC19_MDPE_IK_D278Q was prepared using a plasmid prep kit (GENALL) in substantially the same manner as in Example 2-4.

Additional enzyme mutations were induced in substantially the same manner as the site-directed mutagenesis (SDM) process of Example 3-1, using the prepared plasmid pUC19_MDPE_IK_D278Q as a template, and using the forward primer of SEQ ID NO: 13, 15, or 17 and the reverse primer of SEQ ID NO: 14, 16, or 18 in Table 5, and the primer set in Table 8, and then mutation sites were identified in substantially the same manner as in Example 3-3, thereby obtaining microorganisms with the additional mutations.

**Table 8**

| Type | Nucleotide sequence (5' -> 3') | SEQ ID NO: |
|---|---|---|
| MDPE_P77I_F | CCGACGTTACCTCTTCTGACATTGCGGTTGTT GCGGCGGG | 29 |
| MDPE_P77I_R | CCCGCCGCAACAACCGCAATGTCAGAAGAGG TAACGTCGG | 30 |

(In Table 8, the underlined portions represent the nucleotide sequence portions corresponding to the amino acids to be substituted.)

### Example 5-2. Evaluation of thermal stability of microorganisms producing tertiary mutant enzymes

The half-lives were confirmed for DH10b pUC19_MDPE_IKQ of Example 4 and DH10b pUC19_MDPE_IKQ_P77I, DH10b pUC19_MDPE_IKQ_V132L, DH10b pUC19_MDPE_IKQ_A192T, and DH10b pUC19_MDPE_IKQ_G232E of Example 5-1, which are microorganisms confirmed to have induced P77I, V132L, A192T, or G232E mutation, in substantially the same manner as in Example 4, and the results thereof are shown in Table 9.

The values shown in Table 9 are calculated as percentages (%) of the half-life of each microorganism relative to the half-life of the microorganism DH10b pUC19_MDPE_IKQ at 80 °C, which is taken as 100 %.

**Table 9**

| Strain | Relative thermal stability value (%) |
|---|---|
| DH10b pUC19_MDPE_IKQ | 100 |
| DH10b pUC19_MDPE_IKQ_P77I | 110 |
| DH10b pUC19_MDPE_IKQ_V132L | 200 |
| DH10b pUC19_MDPE_IKQ_A192T | 250 |
| DH10b pUC19_MDPE_IKQ_G232E | 150 |

As a result of measuring the half-lives, it was confirmed that the half-lives of the microorganisms having the additional mutations were increased compared to that of the microorganism in which only A23I, E267K, and D278Q mutations were induced, indicating increased thermal stability.

### Example 6. Construction of tertiary mutant enzyme and evaluation of thermal stability (2)

### Example 6-1. Construction and screening of additional mutants of D-allulose 3-epimerase in microorganism DH10b pUC19_MDPE_IKE

Mutagenesis was performed through site-directed mutagenesis (SDM) using, as a template DNA, the selected MDPE_IKE of Example 4, including an enzyme with excellent thermal stability. In this process, an additional mutation is introduced into the primary mutant, MDPE_IK_S285E, through which the effect of double mutations can be confirmed.

Specifically, for DH10b pUC19_MDPE_IKE prepared in Example 4, the purified and obtained plasmid pUC19_MDPE_IK_S285E was prepared using a plasmid prep kit (GENALL) in substantially the same manner as in Example 2-4.

Additional enzyme mutations were induced using the prepared plasmid pUC19_MDPE_IK_S285E as a template, and using the forward primer of SEQ ID NO: 11 or 19 and the reverse primer of SEQ ID NO: 12 or 20 in Table 5 in substantially the same manner as the site-directed mutagenesis process of Example 3-1, or using the forward primer of SEQ ID NO: 25 and the reverse primer of SEQ ID NO: 26 in Table 6 in substantially the same manner as the site-directed mutagenesis process of Example 3-2, and then mutation sites were identified in substantially the same manner as in Example 3-3, thereby obtaining microorganisms with the additional mutations.

In addition, additional enzyme mutations were induced using, as a template, DNA with an additional mutation I106L using the primers of SEQ ID NOs: 11 and 12 in Table 5, and using the forward primer (the nucleotide sequence of SEQ ID NO: 31 or 33) and the reverse primer (the nucleotide sequence of SEQ ID NO: 32 or 34) in Table 10 in substantially the same manner as the site-directed mutagenesis (SDM) of Example 3-1, or using the forward primer of SEQ ID NO: 25 and the reverse primer of SEQ ID NO: 26 in Table 6 in substantially the same manner as the saturation mutagenesis of Example 3-2, and mutation sites were identified in substantially the same manner as in Example 3-3, thereby obtaining microorganisms with the additional mutations.

**Table 10**

| Type | Nucleotide sequence (5' -> 3') | SEQ ID NO: |
|---|---|---|
| MDPE_S67T_F | GTTTCTGCGTCTCTGGGTCTGACCGGTGCGA CCGACGTTACC | 31 |
| MDPE_S67T_R | GGTAACGTCGGTCGCACCGGTCAGACCCAGA GACGCAGAAAC | 32 |
| MDPE_G68P_F | TCTGCGTCTCTGGGTCTGTCTCCGGCGACCG ACGTTACCTCTTC | 33 |
| MDPE_G68P_R | GAAGAGGTAACGTCGGTCGCCGGAGACAGAC CCAGAGACGCAGA | 34 |

(In Table 10, the underlined portions represent the nucleotide sequence portions corresponding to the amino acids to be substituted.)

### Example 6-2. Evaluation of thermal stability of microorganisms with additional mutations

The half-lives were confirmed for DH10b pUC19_MDPE_IKE of Example 4 and DH10b pUC19_MDPE_IKE_I106L, DH10b pUC19_MDPE_IKE_S142N, DH10b pUC19_MDPE_IKE_D170G, DH10b pUC19_MDPE_IKE_I106L/S67T, DH10b pUC19_MDPE_IKE_I106L/G68P, and DH10b pUC19_MDPE_IKE_I106L/S142N of Example 5-1, which are microorganisms confirmed to have induced I106L, S142N, D170G, I106L/S67T, I106L/G68P, or I106L/S142N mutation, in substantially the same manner as in Example 4, and the results thereof are shown in Table 11.

The values shown in Table 11 are calculated as percentages (%) of the half-life of each microorganism relative to the half-life of the microorganism DH10b pUC19_MDPE_IKE at 75 °C, which is taken as 100 %.

**Table 11**

| Strain | Relative thermal stability value (%) |
|---|---|
| DH10b pUC19_MDPE_IKE | 100 |
| DH10b pUC19_MDPE_IKE_I106L | 160 |
| DH10b pUC19_MDPE_IKE_S142N | 160 |
| DH10b pUC19_MDPE_IKE_D170G | 135 |
| DH10b pUC19_MDPE_IKE_I106L/S67T | 180 |
| DH10b pUC19_MDPE_IKE_I106L/G68P | 220 |
| DH10b pUC19_MDPE_IKE_I106L/S142N | 210 |

As a result of measuring the half-lives, it was confirmed that the half-lives of the microorganisms having the additional mutations were increased compared to the microorganism in which only A23I, E267K, and S285E mutations were induced, indicating increased thermal stability. In particular, it was confirmed that the half-lives of the microorganisms further having the I106L and S68P mutations were significantly increased, demonstrating a significant improvement in thermal stability.

Taking all the thermal stability results into consideration, it was confirmed that DH10b pUC19_MDPE_IKE_I106L/G68P, which is a microorganism having additional S285E, I106L, and S68P mutations in addition to A23I and E267K mutations, exhibited a 700 % or more increase in half-life as compared to that of the microorganism DH10b pUC19_MDPE_IK of Examples 2-4, indicating very high thermal stability.

### Example 7. Evaluation of activity of mutant enzymes with increased thermal stability

Mutant enzymes with enhanced thermal stability via additional mutations were identified in Examples 4 to 6. To compare the enzymatic activities thereof with that of the microorganism having only A23I and E267K mutations, cells of the microorganisms DH10b pUC19_MDPE_IK of Example 2-4, the DH10b pUC19_MDPE_IKQ, DH10b pUC19_MDPE_IKQ_V132L, DH10b pUC19_MDPE_IKQ_A192T, and DH10b pUC19_MDPE_IKQ_G232E of Example 5, and DH10b pUC19_MDPE_IKE, DH10b pUC19_MDPE_IKE_I106L, DH10b pUC19_MDPE_IKE_D170G, DH10b pUC19_MDPE_IKE_I106L/S67T, DH10b pUC19_MDPE_IKE_I106L/G68P, and DH10b pUC19_MDPE_IKE_I106L/S142N of Example 6 were harvested in substantially the same manner as in Example 4.

The cell conversion reaction was performed using the harvested cells at a cell concentration of 1 mg (dcw)/mL in a 50 mM PIPES buffer (pH 6.0) as a solution containing 400 g/L of fructose as a reaction substrate and 1 mM manganese ions, at 70 °C for 30 minutes.

After the conversion reaction, centrifugation and HPLC analysis were performed in substantially the same manner as in Example 4, and the results of the HPLC analysis are shown in Table 12.

The values shown in Table 12 are calculated as percentages (%) of the relative activity value of each microorganism with respect to the substrate conversion activity of the microorganism DH10b pUC19_MDPE_IK, which is taken as 100 %.

**Table 12**

| Strain | Relative activity (%) |
|---|---|
| DH10b pUC19_MDPE_IK | 100 |
| DH10b pUC19_MDPE_IKQ | 110 |
| DH10b pUC19_MDPE_IKQ_V132L | 110 |
| DH10b pUC19_MDPE_IKQ_A192T | 105 |
| DH10b pUC19_MDPE_IKQ_G232E | 115 |
| DH10b pUC19_MDPE_IKE | 110 |
| DH10b pUC19_MDPE_IKE_I106L | 130 |
| DH10b pUC19_MDPE_IKE_D170G | 135 |
| DH10b pUC19_MDPE_IKE_I106L/S67T | 120 |
| DH10b pUC19_MDPE_IKE_I106L/G68P | 170 |

As a result of evaluating enzymatic activity by measuring the substrate conversion activity of the organism, it was confirmed that the activities of microorganisms with additional mutations increased compared to those with the A23I and E267K double mutations. In particular, the activity of the microorganism DH10b pUC19_MDPE_IKE_I106L/G68P, which has additional I106L and S68P mutations, exhibited an increase of 170 % or more compared to the activity of the microorganism DH10b pUC19_MDPE_IK.

## Claims

1. An allulose 3-epimerase protein comprising an amino acid sequence having 80 % or more sequence identity with the amino acid sequence of SEQ ID NO: 1,
wherein, from the N-terminus of the amino acid sequence of SEQ ID NO: 1,
(1) amino acids at positions corresponding to positions 23 and 267 are substituted with isoleucine (I) and lysine (K), respectively, and
(2) one or more amino acids selected from the group consisting of amino acids at positions corresponding to positions 67, 68, 77, 106, 132, 142, 170, 174, 192, 232, 278, and 285 are substituted with one or more amino acids selected from the group consisting of leucine (L), asparagine (N), alanine (A), valine (V), glutamine (Q), glutamic acid (E), isoleucine (I), threonine (T), glycine (G), and proline (P), and
wherein the allulose 3-epimerase protein excludes an allulose 3-epimerase protein comprising an amino acid sequence, in which from the N-terminus of the amino acid sequence of SEQ ID NO: 1, an amino acid at position 23 is substituted with isoleucine (I), and an amino acid at position 267 is substituted with lysine (K).

2. The allulose 3-epimerase protein of claim 1, wherein the allulose 3-epimerase protein comprises, from the N-terminus of the amino acid sequence of SEQ ID NO: 1,
(1) substitution of alanine at a position corresponding to position 23 with isoleucine and substitution of glutamic acid at a position corresponding to position 267 with lysine, and
(2) one or more selected from the group consisting of substitution of serine (S) at a position corresponding to position 67 with threonine, substitution of glycine at a position corresponding to position 68 with proline, substitution of proline at a position corresponding to position 77 with isoleucine, substitution of isoleucine at a position corresponding to position 106 with leucine, substitution of valine at a position corresponding to position 132 with leucine, substitution of serine at a position corresponding to position 142 with asparagine, substitution of serine at a position corresponding to position 142 with alanine, substitution of aspartame at a position corresponding to position 170 with glycine, substitution of leucine at a position corresponding to position 174 with valine, substitution of alanine at a position corresponding to position 192 with threonine, substitution of glycine at a position corresponding to position 232 with glutamic acid, substitution of aspartame at a position corresponding to position 278 with glutamine, substitution of serine at a position corresponding to position 285 with glutamic acid, and substitution of serine at a position corresponding to position 285 with asparagine.

3. The allulose 3-epimerase protein of claim 1, wherein the allulose 3-epimerase protein has a conversion activity of 102 % to 200 % relative to 100 % allulose conversion activity of an enzyme comprising an amino acid sequence having, from the N-terminus of the amino acid sequence of SEQ ID NO: 1, substitution of an amino acid at position 23 with isoleucine (I), and substitution of an amino acid at position 267 with lysine (K).

4. A polynucleotide encoding the allulose 3-epimerase protein of any one of claims 1 to 3.

5. A recombinant vector comprising the polynucleotide according to claim 4.

6. A recombinant microorganism comprising a polynucleotide encoding the allulose 3-epimerase protein of any one of claims 1 to 3 or a recombinant vector comprising the polynucleotide.

7. The recombinant microorganism of claim 6, wherein the microorganism is one or more selected from the group consisting of strains of the genus *Escherichia*, strains of the genus *Bacillus,* strains of the genus *Corynebacterium*, strains of the genus S*accharomyces*, and strains of the genus *Pichia*.

8. A composition for producing allulose, the composition comprising one or more selected from the group consisting of the allulose 3-epimerase protein of any one of claims 1 to 3, a recombinant microorganism expressing the allulose 3-epimerase protein, a cell of the microorganism, a lysate of the cell of the microorganism, a culture of the microorganism, and extracts thereof.

9. The composition for producing allulose of claim 8, wherein a half-life of the allulose 3-epimerase protein is 600 % to 5,000 % under heat treatment conditions of 80 °C, relative to 100 % of the half-life of the allulose 3-epimerase protein comprising the amino acid sequence of SEQ ID NO: 1.

10. The composition for producing allulose of claim 8, wherein a half-life of the allulose 3-epimerase protein is 110 % to 800 % under heat treatment conditions of 80 °C, relative to 100 % of the half-life of the allulose 3-epimerase protein comprising an amino acid sequence comprising, from the N-terminus of the amino acid sequence of SEQ ID NO: 1, substitution of an amino acid at position 23 with isoleucine (I), and substitution of an amino acid at position 267 with lysine (K).

11. The composition for producing allulose of claim 8, wherein the composition further comprises one or more selected from the group consisting of manganese ions, magnesium ions, nickel ions, and cobalt ions.

12. A method of producing allulose, the method comprising reacting, with a substrate, one or more selected from the group consisting of the allulose 3-epimerase protein of any one of claims 1 to 3, a recombinant microorganism expressing the allulose 3-epimerase protein, a cell of the microorganism, a lysate of the cell of the microorganism, a culture of the microorganism, and extracts thereof.
